# EUROPEAN PATENT SPECIFICATION

(11) **EP 2 276 501 B1**
(45) Date of publication and mention of the grant of the patent: **15.02.2017**
(21) Application number: 09719985.5
(22) Date of filing: 20.03.2009
(51) Int. Cl.: A61K 38/16, A61K 38/18, A61K 48/00, A61P 27/02, A61K 39/39

(54) **METHODS AND COMPOSITIONS FOR GENETIC AND RETINAL DISEASE**
VERFAHREN UND ZUSAMMENSETZUNGEN GEGEN GENETISCHE UND NETZHAUTERKRANKUNGEN
PROCÉDÉS ET COMPOSITIONS POUR UNE MALADIE GÉNÉTIQUE ET RÉTINIENNE

(30) Priority: 14.03.2008 US 36717 P
(43) Date of publication of application: 26.01.2011
(73) Proprietor: Drenser, Kimberly, Bloomfield, MI 48302 (US)
(72) Inventor: Drenser, Kimberly, Bloomfield, MI 48302 (US)
(74) Representative: ABG Patentes, S.L.
(86) International application number: PCT/US2009/037792
(87) International publication number: WO 2009/114878

(56) References cited:
- US-A- 5 667 968
- US-A1- 2003 129 164
- PEREZ-VILAR JUAN ET AL: "Norrie disease protein (norrin) forms disulfide-linked oligomers associated with the extracellular matrix", JOURNAL OF BIOLOGICAL CHEMISTRY, AMERICAN SOCIETY FOR BIOCHEMISTRY AND MOLECULAR BIOLOGY, INC, US, vol. 272, no. 52, 26 December 1997 (1997-12-26), pages 33410-33415, XP009089849, ISSN: 0021-9258, DOI: DOI:10.1074/JBC.272.52.33410
- WU WEI-CHI ET AL: "Retinal phenotype-genotype correlation of pediatric patients expressing mutations in the Norrie disease gene.", ARCHIVES OF OPHTHALMOLOGY FEB 2007 LNKD- PUBMED:17296899, vol. 125, no. 2, February 2007 (2007-02), pages 225-230, XP002633256, ISSN: 0003-9950
- OHLMANN, A. ET AL.: 'Ectopic Norrin induces growth of ocular capillaries and restores normal retinal angiogenesis in Norrie disease mutant mice' J. NEUROSCIENCE vol. 25, no. 7, 2005, pages 1701 - 1710, XP002569133
- XU, Q. ET AL.: 'Vascular development in the retina and inner ear: control by Norrin and Frizzled-4, a high-affinity ligand-receptor pair' CELL vol. 116, 2004, pages 883 - 895, XP008133958
- LUHMANN, U. F. O. ET AL.: 'Role of the norrie disease pseudoglioma gene in sprouting angiogenesis during development of the retinal vasculature' INVEST. OPHTHALMOL. VIS. SCI. vol. 46, 2005, pages 3372 - 3382, XP002578265

## Description

### FIELD OF THE INVENTION

The subject invention relates generally to methods and therapies for the treatment of ocular diseases due to acquired retinal or vascular degeneration or genetic abnormality. More specifically, the subject invention relates to treatments and therapeutics to promote vascular and neuronal growth or differentiation in the retinal bed. Most specifically, the subject invention relates to methods and compositions for treatment of familial exudative vitreoretinopathy, or other related vitreoretinal and vascular developmental diseases.

### BACKGROUND OF THE INVENTION

Proper vascular modeling in the retina is essential for ocular development and visual acuity. Abnormal vessel growth during development or in adulthood produces several relatively common diseases such as retinopathy of prematurity, diabetic retinopathy, and age-related macular degeneration. Normal retinal development occurs through vessels forming at the optic nerve head and spreading over the retina to form a dense network. Connolly, SE, et al, Microvasc Res, 1988; 36:275-290; Provis, JM, Prog Retin Eye Res, 2001; 20:799-821; Fruttiger, M, Invest Ophthalmol Vis Sci, 2002; 43:522-527. Development proceeds through formation of primary vessels along the surface of the developing retina from which divergent vessels begin to extend into the capillary beds that form the outer and inner plexiform layers of the retina. Connelly, 1988; Provis, 2001, Fruttiger, 2002. Vascular development is mediated by a series of growth factors that direct formation and extension of new vessels. Retinal development is unique in the concentration and types of signaling mediators employed to promote angiogenic sprouting from the primary vascular network and the formation of the final capillary architecture. Ohlmann, A, et al, J Neurosci, 2005; 25:1701-1710. One factor hypothesized to be involved in formation of primary retinal vasculature and retinal capillaries is the protein Norrin. Norrin, is a 131 amino acid long protein that is secreted into the extracellular space. Meitinger, T, et al, Nat Genet, 1993; 5:376-380; Berger, W, et al, Hum Mol Genet, 1996; 5:51-59. Two primary domains define the general Norrin protein structure: a signal peptide directs localization of the molecule; and a cysteine-knot motif provides the tertiary confirmation required for receptor binding and activation of signal transduction.

The importance of the cysteine knot-motif is highlighted by computer modeling that demonstrates the requirement of disulfide bonds between the cysteine residues in forming the structural confirmation of Norrin. Mutation(s) of the cysteine residues reduces the affinity of Norrin for its receptor and prevents activation of subsequent signaling pathways. Mutations in these residues also result in severe retinal dysgenesis and Norrie disease. However, mutations in regions other than the cysteine knot-motif produce incomplete protein folding and result in familial exudative vitreoretinopathy (FEVR) and related vitreoretinopathies (Retinopathy of Prematurity, persistent fetal vasculature).

Norrin is a ligand for the Frizzled receptor subtype 4 (Fz4). Norrin binds Fz4 with nanomolar affinity and stimulates a Wnt receptor:β-catenin signal transduction pathway that regulates retinal development and is necessary for regression of hyaloid vessels in the eye. Xu, Q, et al, Cell, 2004; 116:883-895; Clevers, H, Curr Biol, 2004; 14:R436-437; Nichrs, C, Dev Cell, 2004; 6:453-454. Norrin interaction with Fz4 is dependent on the cell surface receptor LRP5. Xu, 2004. Frizzled receptors are coupled to the β-catenin canonical signaling pathway that functions by the activation of Wnt target genes. Wnt protein binding to Frizzled and LRP5 inactivates glycogen synthase kinase (GSK) 3ß and Axin. The inactivation of these proteins stabilizes ß-catenin, which subsequently accumulates in the cell nucleus and activates the transduction of target genes that are crucial in the G1-S-phase transition, such as cyclin D1 or c-Myc. Willert K, and Nusse R, Curr Opin Genet Dev, 1998; 8:95-102. These pathways promote stimulation and proliferation of retinal stem cells. Inoue, T, et al, Stem Cells, 2006; 24:95 -104.

Norrin is encoded by the *NDP* gene present on chromosome X at position 11.4. The importance of this gene product is highlighted by observations that inactivating mutations lead to Norrie disease which is characterized by ocular and cochlear vascular defects. Rhem, HL, et al, J Neurosci, 2002; 22:4286-4292; Black, GC, et al, Hum Mol Genet, 1999; 8:2031-2035. Silencing of the NDP gene produces incomplete regression of the primary hyaloid system and abnormal retinal maturation.

Observations that abnormalities in the Fz4 and LRP5 receptors that result in the phenotypically similar condition, FEVR underscore the importance of Norrin signaling. Robitaille, J, et al, Nature Genet, 2002; 32:326-330; Kondo, H, et al, Br J Opthalmol, 2003; 87:1291-1295; Toomes, C, et al, Am J Hum Genet, 2004; 74:721-730. The close association between the phenotypes produced by Norrin mutations and mutations in the Fz4 and LRP5 receptors bolsters the hypothesis that these molecules form a functional signaling group. Planutis, K, et al, BMC Cell Biology, 2007; 8:12.

While defects in the NDP gene and diseases due to incomplete or immature vascularization have been studied and correlated with disease, therapies presently available for Norrie disease, FEVR, or other retinal diseases are only modestly effective. Thus, there exists a need for improved therapeutics and methods of treatment for vitreoretinal disease and vascular disease in the retina.

### SUMMARY OF THE INVENTION

The present invention provides a Norrin compound for use in the treatment of a pathological condition of the retina, wherein the Norrin compound is a nucleotide sequence encoding a Norrin protein, a cell transfected with a nucleotide sequence encoding a Norrin protein and comprising said nucleotide sequence, or a Norrin protein wherein the Norrin protein is selected from the group consisting of a polypeptide having the sequence of SEQ ID NO: 1, SEQ ID NO: 2, SEQ ID NO: 3, an oligomeric complex comprising a Norrin protein, and a mutant form of Norrin protein, wherein the mutant form is selected from the group of consisting of the R64E mutant, the T27A, S28A and S29A mutant; the P36A, R37A and R38A mutant and the H127A, E129A and E130A mutant of the polypeptide of SEQ ID NO:1, wherein the retinal disease is selected from the group consisting of vitreoretinopathy, retinopathy of prematurity, and familial exudative vitreoretinopathy.

### BRIEF DESCRIPTION OF THE DRAWINGS

**Figure 1** depicts protection against retinal damage by NDMA *in vivo;*
**Figure 2** depicts growth and differentiation of neural progenitor cells in *in vivo* retinas following administration of Norrin;
**Figure 3** depicts early development of vascular channels during development of stem cells into embroyid bodies due to the presence of Norrin;

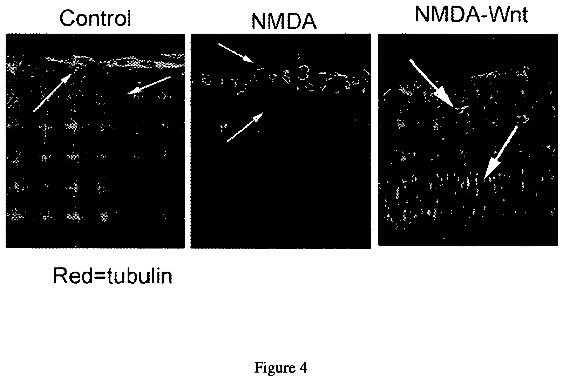

**Figure 4** depicts *in vivo* retinal cell differentiation and protection from NDMA damage by activation of the Wnt pathways;

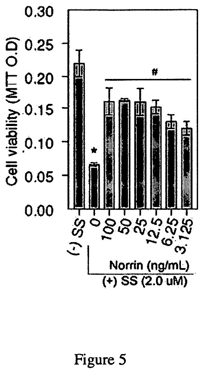

**Figure 5** depicts subsequent art illustrating protection of RGC5 cell viability from staurosporine damage by various concentrations of Norrin. Lin, S, et al, Molecular Vision 2009.

### DETAILED DESCRIPTION OF THE PREFERRED EMBODIMENTS

It is to be understood that the present invention is not limited to particular embodiments described, as such may, of course, vary. It is also to be understood that the terminology used herein is for the purpose of describing particular embodiments only, and is not intended to be limiting.

The present invention provides a Norrin compound for use in the treatment of a pathological condition of the retina, wherein the Norrin compound is a nucleotide sequence encoding a Norrin protein, a cell transfected with a nucleotide sequence encoding a Norrin protein and comprising said nucleotide sequence, or a Norrin protein wherein the Norrin protein is selected from the group consisting of a polypeptide having the sequence of SEQ ID NO: 1, SEQ ID NO: 2, SEQ ID NO: 3, an oligomeric complex comprising a Norrin protein, and a mutant form of Norrin protein, wherein the mutant form is selected from the group of consisting of the R64E mutant, the T27A, S28A and S29A mutant; the P36A, R37A and R38A mutant and the H127A, E129A and E130A mutant of the polypeptide of SEQ ID NO:1, wherein the retinal disease is selected from the group consisting of vitreoretinopathy, retinopathy of prematurity, and familial exudative vitreoretinopathy.

Thus, the subject invention has utility for the prevention, reversal, or treatment of vitreoretinal and vascular disease.

As used herein, the term "subject" illustratively includes a mammal, human, cow, horse, sheep, pig, goat, chicken, cat, dog, mouse, guinea pig, hamster, rabbit, rat, a cell, tissue, organ, organ system, or combinations thereof. It is appreciated that the term subject is optionally a patient.

As used herein, the terms "pathological condition" or "disease" illustratively include vitreoretinopathy, retinopathy of prematurity (retrolental fibroplasias), Familial Exudative Vitreoretinopathy (FEVR), Norrie disease, Persistent Fetal Vasculature Syndrome (persistent hyperplastic primary vitreous), Coats disease, Macular Degeneration, Macular Dystrophy, inherited dystrophy, cancer, growth of abnormal cells, Osteoporosis-Pseudoglioma Syndrome, Non-Exudative Age Related Macular Degeneration (ARMD), Exudative Age Related Macular Degeneration (ARMD), Choroidal Neovascularization, Diabetic Retinopathy, Retinal Vein Occlussion, Retinal Artery Occlussion, Acute Macular Neuroretinopathy, Central Serous Chorioretinopathy, Cystoid Macular Edema, Diabetic Macular Edema, Acute Multifocal Placoid Pigment Epitheliopathy, Behcet's Disease, Birdshot Retinochoroidopathy, Infectious (Syphilis, Lyme, Tuberculosis, Toxoplasmosis), Intermediate Uveitis (Pars Planitis), Multifocal Choroiditis, Multiple Evanescent White Dot Syndrome (MEWDS), Ocular Sarcoidosis, Posterior Scleritis, Serpignous Choroiditis, Subretinal Fibrosis and Uveitis Syndrome, Vogt-Koyanagi-Harada Syndrome, Parafoveal Telangiectasis, Papillophlebitis, Frosted Branch Angitis, Sickle Cell Retinopathy and other Hemoglobinopathies, Angioid Streaks, Sympathetic Ophthalmia, Uveitic Retinal Disease, Retinal Detachment, Trauma, Laser, PDT, Photocoagulation, Hypoperfusion During Surgery, Radiation Retinopathy, Bone Marrow Transplant Retinopathy, Proliferative Vitreal Retinopathy and Epiretinal Membranes, Proliferative Diabetic Retinopathy, , Ocular Histoplasmosis, Ocular Toxocariasis, Presumed Ocular Histoplasmosis Syndrome (POHS), Endophthalmitis, Toxoplasmosis, Retinal Diseases Associated with HIV Infection, Choroidal Disease Associated with HIV Infection, Uveitic Disease associated with HIV Infection, Viral Retinitis, Acute Retinal Necrosis, Progressive Outer Retinal Necrosis, Fungal Retinal Diseases, Ocular Syphilis, Ocular Tuberculosis, Diffuse Unilateral Subacute Neuroretinitis, Myiasis, Systemic Disorders with Associated Retinal Dystrophies, Congenital Stationary Night Blindness, Cone Dystrophies, Fundus Flavimaculatus, Best's Disease, Pattern Dystrophy of the Retinal Pigmented Epithelium, X-Linked Retinoschisis, Sorsby's Fundus Dystrophy, Benign Concentric Maculopathy, Bietti's Crystalline Dystrophy, pseudoxanthoma elasticum, Osler Weber syndrome, Retinal Detachment, Macular Hole, Giant Retinal Tear, Retinal Disease Associated with Tumors, Solid Tumors, Tumor Metastasis, Benign Tumors, for example, hemangiomas, neurofibromas, trachomas, and pyogenic granulomas, Congenital Hypertrophy of the RPE, Posterior Uveal Melanoma, Choroidal Hemangioma, Choroidal Osteoma, Choroidal Metastasis, Combined Hamartoma of the Retina and Retinal Pigmented Epithelium, Retinoblastoma, Vasoproliferative Tumors of the Ocular Fundus, Retinal Astrocytoma, Intraocular Lymphoid Tumors, Punctate Inner Choroidopathy, Acute Posterior Multifocal Placoid Pigment Epitheliopathy, Myopic Retinal Degeneration, Acute Retinal Pigment Epithelitis, Ocular inflammatory and immune disorders, ocular vascular malfunctions, Corneal Graft Rejection, Neovascular Glaucoma, and other diseases of ocular vascular development.

As used herein, the term "physiological activity" illustratively includes vascularization, cell proliferation, cellular interaction, cellular differentiation, reduction in apoptosis, reduction in necrosis, neuroprotection, growth, vascular regression, CAMKII phosphorylation, protein kinase C (PKC) activation, protein kinase A activation, activation of the MAPK pathway (illustratively, MAPK8 or JNK), downstream gene activation, activation of the β-catenin pathway, cellular viability, proteolytic activity, phosphorylation, dephosphorylation, receptor activation, receptor inactivation, other activities illustratively describe by Lin, S, et al, Molecular Vision 2009; 15:26-37, or combinations thereof.

As used herein, the term "cell" illustratively includes a somatic cell, a germ cell, a progenitor cell, a cultured cell, a stem cell, a transfected cell, or combinations thereof.

As used herein, the term "administering" illustratively includes delivery of a molecule or a cell to a subject by a route illustratively including systemic administration, local administration, injection, intravitreal injection, subconjuctival injection, sub-tenon injection, retrobulbar injection, suprachoroidal injection, surgical implantation, topical administration, iontophoretic delivery, oral, rectal, parenteral, intravenous, intramuscular, subcutaneous, intracisternal, intravaginal, intraperitoneal, intravesical, intraventricular, intracranial, intratumoral, local, transdermal, intrabuccal, intranasal, intrathecal, modifications thereof, or combinations thereof.

As used herein a "compound" is illustratively a protein, DNA, RNA, lipid, steroid, growth factor, antibody, antibody fragment, Fab', F(ab)₂, Fabc, Fv fragment, organic molecule, a cell, fragments thereof, mutations thereof, or mimics thereof. A compound is optionally recombinant. Most preferably, a compound encodes Norrin such as a Norrin compound.

As used herein, the term "stem cell" is illustratively a cell possessing self-replicating potential and the ability to give rise to terminally differentiated cells of single or multiple lineages. Stem cells are capable of generating identical progeny through unlimited numbers of cell divisions while retaining the ability to respond to physiological demands by producing daughters committed to differentiate.

Antibodies useful in the present systems include antibody fragments, such as Fab', F(ab)₂, Fabc, and Fv fragments. Antibody fragments are optionally produced by modification of whole antibodies or synthesized *de novo* using recombinant DNA methodologies, and further include "humanized" antibodies made by conventional or nonconventional techniques.

It is disclosed a compound administered herein which is optionally supplemented with one or more agents effective in reducing inflammation, reducing pain, reducing or preventing tumor growth, reducing intraocular pressure, protecting cells, such as retinal neurons, reducing excitotoxicity, reducing infection, and reducing hemorrhage. A co-administered agent is optionally cytotoxic depending on the condition being treated. In addition, a co-administered agent optionally comprises a neurotoxic macromolecule, such as a botulinum neurotoxin, in combination with the non-neurotoxic macromolecule. A co-administered agent optionally comprises a small chemical compound in combination with the present macromolecules. Examples of chemical compounds operable herein illustratively include a small chemical compound, such as anecortave acetate, ketorlac tromethamine (such as Acular), gatifloxacin, ofloxacin, epinastine, and the like.

Optionally, a compound is delivered to a subject conjugated to a magnetic particle as described in U.S. Patent Application Publication 2004/0086572.

A Norrin compound is a nucleotide sequence or a protein sequence encoding a Norrin protein. A Norrin compound is illustratively SEQ ID NOs: 1-6. A Norrin compound is optionally purified.

In a preferred embodiment Norrin protein is administered to a subject. Norrin is optionally purified from a subject or expressed in a recombinant cell system and subsequently purified. Purification is not absolutely required but is preferred. Norrin protein sequence operable herein illustratively includes that derived from human, mouse, rat, dog, cat, or other suitable organism. SEQ ID NO: 1 represents the Norrin sequence from homo sapiens. SEQ ID NO: 2 represents the Norrin sequence from Mus musclus. SEQ ID NO: 3 represents the Norrin sequence from Rattus norvegicus.

A Norrin protein is optionally administered as an oligomeric complex. See Perez-Vilar, J and Hill, RL, J Biol Chem, 1997; 272: 33410-33415. Oligomers operable herein are optionally dimers, trimers, quadramers, pentamers, hexamers, octamers, or higher order multimers. Oligomeric complexes are optionally homomultimeric or heteromultimeric. The range of oligomeric association is preferably between monomers and 30-mers. More preferably, the oligomeric association is between dimers and 20-mers. Most preferably, the oligomeric association is monomers or dimers.

Norrin is optionally administered to a subject with a cofactor. A cofactor is illustratively a polyanion. More preferably a cofactor is heparin or heparin sulfate. It is appreciated that other polyanions or modifications thereof are similarly operable.

A Norrin protein is preferably administered to a subject at a concentration suitable to alter or maintain a physiological activity. The total quantity or concentration of Norrin protein delivered is optionally dependent on the age or size of the subject as appreciated by one of skill in the art.

It is disclosed a compound which is a biologically active polypeptide fragment of Norrin protein which is administered to a subject. A biologically active polypeptide fragment illustratively includes residues 20-133 of SEQ ID NO: 1, Residues 30-133 of SEQ ID NO: 1, or any other truncation N-terminal to cysteine 39 of SEQ ID NO: 1 and extending toward the C-terminus of Norrin protein. It is appreciated that other polypeptide regions of the Norrin protein or nucleotide sequence will be similarly operable.

A biologically active peptide optionally is a mutant form of Norrin. Norrin mutants operable herein illustratively include amino acid substitutions relative to SEQ ID NO: 1 of R64E. Optionally the biologically active peptide is a multiple mutant relative to SEQ ID NO: 1: T27A, S28A, S29A; P36A, R37A, R38A; or H127A, E129A, E130A; or combinations thereof. Any amino acid mutated in a multiple mutation is operable as a single mutation. Other sequence mutations operative herein are illustrated in figure 6A of Smallwood, PM, et al, J Biol Chem, 2007: 282:4057-4068. It is appreciated that other mutations at different amino acid sites are similarly operable. It is further appreciated that mutation of the conserved amino acid at any particular site is preferably mutatated to glycine or alanine. It is further appreciated that mutation to any neutrally charged, charged, hydrophobic, hydrophilic, synthetic, non-natural, non-human, or other amino acid is similarly operable.

In one aspect, the document discloses modifications and changes which are optionally made in the structure (primary, secondary, or tertiary) of the Norrin protein which may or may not result in a molecule having similar characteristics to the exemplary polypeptides disclosed herein. It is appreciated that changes in conserved amino acid bases are most likely to impact the activity of the resultant protein. However, it is further appreciated that changes in amino acids operable for receptor interaction, resistance or promotion of protein degradation, intracellular or extracellular trafficking, secretion, protein-protein interaction, post-translational modification such as glycosylation, phosphorylation, sulfation, and the like, may result in increased or decreased activity of an inventive compound while retaining some ability to alter or maintain a physiological activity. Certain amino acid substitutions for other amino acids in a sequence are known to occur without appreciable loss of activity.

In making such changes, the hydropathic index of amino acids are considered. According to the present invention, certain amino acids can be substituted for other amino acids having a similar hydropathic index and still result in a polypeptide with similar biological activity. Each amino acid is assigned a hydropathic index on the basis of its hydrophobicity and charge characteristics. Those indices are: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

Without intending to be limited to a particular theory, it is believed that the relative hydropathic character of the amino acid determines the secondary structure of the resultant polypeptide, which in turn defines the interaction of the polypeptide with other molecules. It is known in the art that an amino acid can be substituted by another amino acid having a similar hydropathic index and still obtain a functionally equivalent polypeptide. In such changes, the substitution of amino acids whose hydropathic indices are within ± 2 is preferred, those within ± 1 are particularly preferred, and those within ±0.5 are even more particularly preferred.

As outlined above, amino acid substitutions are generally based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include (original residue: exemplary substitution): (Ala: Gly, Ser), (Arg: Lys), (Asn: Gln, His), (Asp: Glu, Cys, Ser), (Gln: Asn), (Glu: Asp), (Gly: Ala), (His: Asn, Gln), (Ile: Leu, Val), (Leu: Ile, Val), (Lys: Arg), (Met: Leu, Tyr), (Ser: Thr), (Thr: Ser), (Tip: Tyr), (Tyr: Trp, Phe), and (Val: Ile, Leu). Embodiments of this disclosure thus contemplate functional or biological equivalents of a polypeptide as set forth above. In particular, embodiments of the polypeptides can include variants having about 50%, 60%, 70%, 80%, 90%, and 95% sequence identity to the polypeptide of interest.

In one aspect, it is disclosed a Norrin mimetic. A Norrin mimetic is illustratively a polypeptide, lipid, DNA structure, RNA structure, small molecule, other molecule optionally capable of interacting with a Fz receptor, fragments thereof, synthetic analogous thereof, or combinations thereof. Preferably a Norrin mimetic is a molecule capable of stimulating signaling via the Fz4 receptor. Norrin mimetics operable herein are illustratively a WNT protein, a mutation of a WNT protein, antibodies, antibody fragments, or combinations thereof. It is appreciated that other molecules known in the art to stimulate signaling via Fz4 are similarly operable.

Multiple physiological activities are altered or maintained in the present inventive method. Physiological activities illustratively include vascularization, cell proliferation, cellular interaction, neuroprotection, growth, vascular regression, b-wave response, substantial oscillatory potential, or combinations thereof.

In a preferred embodiment Norrin is administered to a subject as a factor suitable for expression within the eye or within a cell located in the eye. An exemplary system for the expression of protein is described in U.S. Patent Application Publication 2003/0129164. Any of a variety of vectors adapted for expression of Norrin in a cell of the eye, preferably within a retinal cell, are within the scope of the present invention. Gene delivery vectors are optionally viral (e.g., derived from or containing sequences of viral DNA or RNA, preferably packaged within a viral particle), or non-viral (e.g., not packaged within a viral particle, including "naked" polynucleotides, nucleic acid associated with a carrier particle such as a liposome or targeting molecule, and the like).

The viral factor operable herein is illustratively a recombinant adeno-associated viral (rAAV) vector. It is appreciated the other suitable vectors known in the art are similarly operative. Additional illustrative vectors optionally include adenoviral vectors, alphaviral vectors, viruses illustratively including pox viruses- illustratively canary pox virus or vaccinia virus, SV40, influenza virus, HIV, herpes, measles, Semliki Forest Virus, and coronavirus, as well as other viral systems. In addition, viral carriers are optionally homologous, non-pathogenic (defective), replication competent virus.

A particularly preferred gene delivery vector is a rAAV vector. A variety of rAAV vectors are optionally utilized to direct the expression of a neurotrophic factor such as Norrin. An operable rAAV is generally comprised of, in order of 5' to 3', a 5' adeno-associated virus inverted terminal repeat, a coding sequence for the desired gene product (e.g., Norrin) operatively linked to a sequence which regulates its expression in a cell (e.g., a promoter sequence), and a 3' adeno-associated virus inverted terminal repeat. A promoter sequence is preferably a cell specific promoter sequence. In addition, the rAAV vector preferably has a polyadenylation sequence. The promoter is illustratively a constitutive promoter, a cell specific promoter, a selective molecule responsive promoter, or combinations thereof. Preferably a promoter is a cell specific promoter. More preferably, a promoter is a constitutive promoter that is cell type specific. Promoter sequences operative herein illustratively include the GFAP promoter, a retinal pigment cell specific promoter, a Muller cell specific promoter, promoters described in WO/2000/015822, a Cdc6 promoter, human ICAM-2 promoter, promoters and vectors described by Dai, C, et al, J Virology, 2004; 78:6209-6221, or other promoter known in the art.

Norrin is optionally delivered to the eye by one or multiple routes illustratively including intraocularly, by topical application to the eye or by intraocular injection illustratively into the vitreous or subretinal (interphotoreceptor) space. Alternatively, delivery is local by insertion or injection into the tissue surrounding the eye, systemically through an oral route, or by subcutaneous, intravenous or intramuscular injection. Alternatively, delivery is by a catheter or by means of an implant, wherein such an implant is made of a porous, non-porous or gelatinous material, including membranes such as silastic membranes or fibers, biodegradable polymers, or proteinaceous material. A compound is illustratively administered prior to the onset of a pathological condition to prevent its occurrence, for example, during or prior to surgery on the eye, or immediately after the onset of the pathological condition or during the occurrence of an acute or protracted disease.

In a preferred embodiment, a compound is administered to the eye, preferably intraocularly to a variety of locations within the eye depending on the type of disease to be treated, prevented, or, inhibited, and the extent of disease. Examples of suitable locations include the retina (e.g., for retinal diseases), the vitreous, or other locations in or adjacent the retina or in or adjacent to the eye.

In one aspect a cell is transfected with a nucleotide sequence encoding a Norrin protein and comprising said nucleotide sequence. Preferably, a nucleotide sequence is cloned into an expression vector with a promoter. The promoter is illustratively a constitutive promoter, strong promoter (e.g., CMV promoters), inducible promoter, tissue-specific promoter, a cell specific promoter, a selective molecule responsive promoter, or combinations thereof. Preferably a promoter is a cell specific promoter. More preferably, a promoter is a constitutive promoter that is cell type specific. Promoter sequences operative herein illustratively include the GFAP promoter, a retinal pigment cell specific promoter, a Muller cell specific promoter, promoters described in WO/2000/015822, a Cdc6 promoter, human ICAM-2 promoter, promoters and vectors described by Dai, C, et al, J Virology, 2004; 78:6209-6221, or other promoter known in the art. In a preferred embodiment a tetracycline responsive promoter is used to limit expression to instances when tetracycline is present in the system.

Numerous enhancers are operable to stimulate expression of Norrin in a transfected cell. Illustratively an endothelial enhancer such as that described by Shaw, LC, et al, Gene Therapy, 2006; 13:752-760 is operative herein.

A cell is preferably a mammalian cell. A cell is optionally an endothelial cell, vascular cell, stem cell, immortalized cell, or combinations thereof. Preferably, a cell is a stem cell. It is appreciated that a stem cell illustratively includes a primary stem cells and lineage cells.

In a preferred embodiment, the cell is a stem cell. A stem cell operable herein and its method of isolation and preparation is illustratively described in U.S. Patent Application Publication 2007/0154465 A1. Stem cells are illustratively transfected with a vector encoding and inventive compound. Suitable vectors and methods of administration to a stem cell are also illustrated in U.S. Patent Application Publication 2007/0154465 A1. Stem cells are optionally transiently transfected or transfected with a vector that provides incorporation into the host genome and constitutive expression.

In a preferred embodiment, a cell is transfected with an expression vector encoding a Norrin protein, and the transfected cell is administered to a subject. An advantage to this embodiment is that possible barriers to optimal protein expression from host cells are overcome by capitalizing on known techniques of in vitro cell transfection. As such, delivery of Norrin protein to the retina of a subject is greatly simplified and expression is improved or maximized. Further, when endothelial cell, stem cell capable of differentiating into an endothelial cell, or other suitable cell type is used as the transfected cell, its subsequent administration to the eye of a subject provides suitable endogenous growth mediators such that cell survival is optimal and duration of successful delivery of a compound is maximized in concentration and time.

Optionally, a cell is derived from a patient. Illustratively, a stem cell is obtained from the patient, transfected, and administered to the patient as a Norrin expressing cell. In a preferred embodiment a retinal cell is obtained from a subject, is transfected with an expression vector expressing an inventive compound, and the transfected cell is administered to the subject.

Administration to the ocular environment in a subject is most preferably by injection into the vitreous. While patients who suffer from a pathological condition of the retina are amenable to multiple injections over the course of time, the use of transfected cells expressing a compound will preferably reduce the number of injections a subject must endure. Illustratively, a single injection of endothelial cells that have been transfected with a vector expressing an inventive compound onto the retina of a subject provides a long-term delivery of the inventive compound to the subject's retina. As such, a single therapeutic delivery will produce a clinical benefit.

In one aspect, it is disclosed that the instant inventive Norrin compound is employed with a subject that has a pathological condition. Pathological conditions are preferably vitreoretinopathy, retinopathy of prematurity, familial exudative vitreoretinopathy. In different aspects, Norrie disease, persistent fetal vasculature syndrome, Coats disease, macular degeneration, macular dystrophy, inherited dystrophy, cancer, growth of abnormal cells, osteoporosis-pseudoglioma syndrome, and other diseases of ocular vascular and retinal development are also disclosed.

Preferably, a pathological condition results from, is caused by, is related to, or otherwise is associated with a mutation in the NDP gene, alteration in transcription to mRNA, mRNA survival, mRNA degradation, alternative mRNA splicing, protein translation, protein survival, protein degradation, protein trafficking, expression of protein on a cell membrane, protein secretion, or other abnormality in production of a functional Norrin protein. This includes decreased production of Norrin secondary to alternate gene expression or suppressed gene activation, seen in mature tissues. Most preferably, a pathological condition is associated with a mutation in the Norrin protein. In a most preferred embodiment a pathological condition is a pathological condition of the retina.

In a most preferred embodiment a Norrin compound is administered to a subject with a pathological condition and the presence of the compound alters or maintains at least one physiological activity. A physiological activity is preferably observed in the retina of a subject. This embodiment has the greatest efficacy in patients suffering from diseases that manifest themselves during development and childhood such as FEVR or Norrie disease. Further, patients illustratively presenting with age related macular degeneration or young patients presenting with early stage disease will benefit from maintenance of retinal vascularization, oxygenation, or visual acuity. The instant inventive method is operable to reduce the rate of visual or other vascular related degeneration relative to subjects not receiving treatment.

A function of the retina is illustratively vascularization, cell proliferation, cellular interaction, neuroprotection, growth, vascular regression, b-wave response, substantial oscillatory potential, Wnt signaling, Fz signaling, CAMKII autophosphorylation, PKC activation, protein kinase A activation, activation of the MAPK pathway , stimulation of gene transcription, activation of intracellular signaling pathways illustratively including the PI3K/Akt pathway, preventing nuclear translocation of c-Jun N-terminal protein (JNK), or regulating caspase activation, stimulation of marker signaling, inhibition of marker signaling, maintenance of marker signaling, or combinations thereof. In a preferred embodiment retinal vascularization is increased. Most preferably, retinal vascularization approaches that observed in subjects that do not present with a pathological condition of the retina. It is appreciated that other markers of proper vascularization are also a function of the retina. These illustratively include development or differentiation of neuronal cells, or activity of neuronal cells.

A marker is illustratively a component of a compound. Illustratively, a cell is transfected with an expression vector that encodes a marker as well as a vector that encodes a compound. The administration of a marker provides the advantage of monitoring the function of the inventive method in cells or in a subject to whom a compound is administered. Markers operable herein are illustratively green fluorescent protein, luciferase, and β-galactosidase. It is appreciated that other suitable markers known in the art are similarly operable. A marker is optionally radioactive, luminescent, biologically active, or otherwise amenable to detection by methods known in the art.

Methods involving conventional biological techniques are described herein. Such techniques are generally known in the art and are described in detail in methodology treatises such as Molecular Cloning: A Laboratory Manual, 2nd ed., vol. 1-3, ed. Sambrook et al., Cold Spring Harbor Laboratory Press, Cold Spring Harbor, N.Y., 1989; and Current Protocols in Molecular Biology, ed. Ausubel et al., Greene Publishing and Wiley-Interscience, New York, 1992 (with periodic updates). Immunological methods (e.g., preparation of antigen-specific antibodies, immunoprecipitation, and immunoblotting) are described, e.g., in Current Protocols in Immunology, ed. Coligan et al., John Wiley & Sons, New York, 1991; and Methods of Immunological Analysis, ed. Masseyeff et al., John Wiley & Sons, New York, 1992.

Various aspects of the present invention are illustrated by the following non-limiting examples. The examples are for illustrative purposes and are not a limitation on any practice of the present invention. While the examples are generally directed to mammalian tissue, specifically, analyses of murine tissue, a person having ordinary skill in the art recognizes that similar techniques and other techniques known in the art readily translate the examples to other organisms such as humans. Reagents illustrated herein are commonly cross reactive between mammalian species or alternative reagents with similar properties are commercially available, and a person of ordinary skill in the art readily understands where such reagents may be obtained.

### Example 1

Preparation of Norrin containing microspheres. Norrin purchased from R&D Systems, Minneapolis, MN, or cloned, expressed and purified is loaded into biodegradable microspheres substantially as described by Jiang, C, et al, Mol Vis, 2007; 13:1783-92 using the spontaneous emulsification technique of Fu, K, et al, J Pharm Sci, 2003; 92:1582-91. Microspheres are synthesized and loaded by dissolving 200 mg of 50:50 PLGA (DURECT Corp., Birmingham, AL) in 5 ml of 4:1 volume ratio trifluoroethanol:dichloromethane supplemented with 8 mg magnesium hydroxide to minimize protein aggregation during encapsulation. 10 µg Norrin is reconstituted in 300 µl 7 mg bovine serum albumin (BSA) and 100 mg docusate sodium (Sigma-Aldrich, St. Louis, MO) dissolved in 3 ml PBS. The solution is vortexed and poured into 200 ml of 1% (w/v) polyvinyl alcohol (PVA, 88% hydrolyzed) with gentle stirring. Microspheres are hardened by stirring for three hours, collected by centrifugation, and washed three times to remove residual PVA. If the microspheres are not to be immediately injected they are rapidly frozen in liquid nitrogen, lyophilized for 72 h, and stored in a dessicator at -20 °C. Norrin containing microspheres exhibit average diameters of 8 µm as determined by a particle size.

### Example 2

Intravitreal injection of Norrin. Norrin in solution, packaged into microspheres as described in Example 1, or expressed in cells, or in purified form in solution is exposed to the retina by intravitreal injection substantially as described by Jiang, 2007. Intravitreal injection is performed under general anesthesia using an ophthalmic operating microscope (Möller-Wedel GmbH, Wedel, Germany) using beveled glass micro-needles with an outer diameter of approximately 100 µm. Microsphere suspensions are prepared in PBS at 2 and 10% (w/v) and briefly vortexed immediately before injection to ensure a uniform dispersion. A 30-gauge hypodermic needle is used to perforate the sclera 1.5 mm behind the limbus. Five µl of test sample is optionally injected by way of this passage into the vitreous using a 50 µl Hamilton Syringe (Hamilton Co, Reno, NV). To ensure adequate delivery and prevent shock the needle is held in place for one min after the injection is completed and subsequently withdrawn slowly. In addition, paracentesis is simultaneously performed to relieve pressure and thereby prevent reflux.

### Example 3

Delivery of Norrin to the retina by a controlled release delivery system. An implantable controlled release delivery system is described in U.S. Patent Application Publication 2005/0281861 A1. Norrin is packaged into such a system at 100 µg per final formulated capsule. The Norrin containing drug delivery systems are placed in the eye using forceps or a trocar after making a 2-3 mm incision in the sclera. Alternatively, no incision is made and the system is placed in an eye by inserting a trocar or other delivery device directly through the eye. The removal of the device after the placement of the system in the eye can result in a self-sealing opening. One example of a device that is used to insert the implants into an eye is disclosed in U.S. Patent Application Publication No. 2004/0054374. The location of the system may influence the concentration gradients of therapeutic component or drug surrounding the element, and thus influence the release rates (e.g., an element placed closer to the edge of the vitreous may result in a slower release rate). Thus, it is preferred if the system is placed near the retinal surface or in the posterior portion of the vitreous.

### Example 4

Treatment of retinal explants with Norrin. Retinal explants from rats or mice are obtained and cultured as described. Nakhai, H, et al, Development, 2007; 134:1151-1160. Eyes are isolated from E18.5 embryos. Explants of the neural retina are dissected and placed into a Millicell CM chamber (Millipore) with the ganglion cell layer upward and cultured in 50% DMEM (Gibco) supplemented with HEPES, 25% Hank's solution, 25% heat-inactivated horse serum, 200 µM L-glutamine and 6.75 mg/ml glucose at 34°C in a 5% CO₂ incubator.

### Example 5

Administered Norrin is protective of NDMA induced retinal damage. Eighteen adult male ddY mice weighing 36 to 43 g each are anesthetized with 3.0% isoflurane and maintained with 1.5% isoflurane in 70% N₂O and 30% O₂. Body temperatures are maintained between 37.0°C and 37.5°C with the aid of a heating pad and a heating lamp. One eye of each animal is designated a control eye with the other eye used as a test eye. At day 0 all test eyes and ½ of control eyes are injected into the vitreous body with n-methyl D-aspartate (NMDA) (Sigma-Aldrich) at 200 nM per eye dissolved in 0.01 M phosphate-buffered saline (PBS) to induce retinal damage. The remaining control eyes are injected with an equal volume of PBS. At day 1, test eyes are injected with either Wnt3 or Norrin as prepared in example 1 at concentrations of 0.1, 1, or 10 ng per eye. One drop of levofloxacin ophthalmic solution (Santen Pharmaceuticals Co. Ltd., Osaka, Japan) is applied topically to each eye after intravitreous injection. Animals are returned to cages and maintained until day 6 at which time they are sacrificed and all eyes are enucleated for retinal tissue fixation and immunohistochemistry. Eyes are enucleated, fixed in 4% paraformaldehyde overnight at 4°C, immersed in 20% sucrose for 48 hours at 4°C, and embedded in optimum cutting temperature (OCT) compound (Sakura Finetechnical Co., Ltd., Tokyo, Japan). Transverse, 10-µm-thick cryostat sections are cut and placed onto slides (Mas Coat). Sections are subsequently processed for immunocytochemistry following staining for Glutamine synthatase and staining for DNA using DAPI.

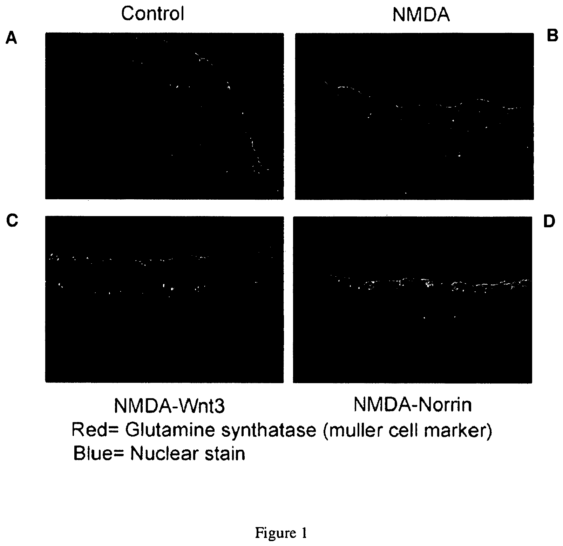

NMDA treated eyes develop numerous lesions indicative of neuronal damage (FIG. 1B). Treatment with positive control Wnt3 illustrates much less retinal damage (FIG. 1C). Treatment of mouse eyes with norrin is protective of damage as illustrated by nearly complete protection against NMDA damage as compared to control eyes (FIG. 1D).

### Example 6

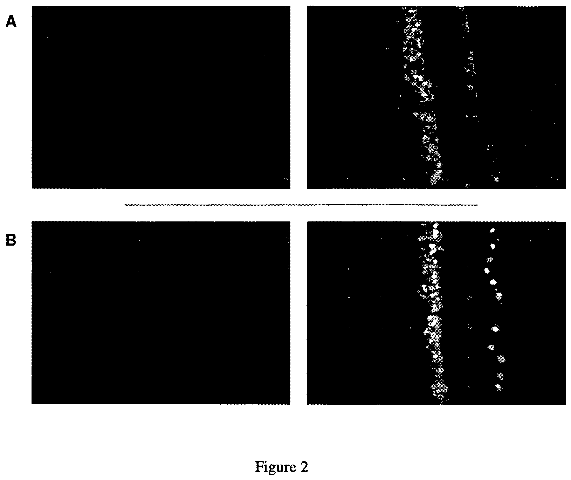

Norrin promotes retinal progenitor cell differentiation in NMDA damages eyes. Mouse eyes are treated with PBS, NMDA, or NMDA plus Norrin as described in example 5. Retinal samples are stained with either Chx10 or Pax6. Costaining with both Chx10 (red) and Pax6 (green) is indicative of the presence of retinal progenitor cells. Norrin treatment (FIG. 2B) results in increased numbers of retinal progenitor cells as compared to control non-norrin treated eyes (FIG. 2A).

### Example 7

Norrin produces earlier development of vascular channels in developing stem cells.

Endothelial stem cells (EC cells) are differentiated and analyzed essentially as described by Ng, Y, et al. Laboratory Investigation, 2004; 84:1209-1218. Briefly, trypsinized ES cells are suspended in culture medium including high-glucose Dulbecco's modified Eagle's medium (DMEM, GIBCO BRL, Grand Island, NY, USA) with 15% fetal bovine serum (Hyclone, UT, USA), sodium pyruvate (GIBCO, stock solution diluted 1:100), nonessential amino acids (GIBCO, stock solution diluted 1:100), β-mercaptoethanol (GIBCO, final concentration 30 µM), 190 µg/ml of L-glutamine, 60 U/ml of penicillin G, 60 µg/ml of streptomycin (glutamine pen-strep mix, Irvine Scientific, Santa Ana, CA, USA), supplemented with media (1:300 dilution) conditioned by Chinese hamster ovary cells overexpressing LIF (provided by Genetics Institute, Cambridge, MA, USA) as a source of LIF to maintain the ES cells in an undifferentiated state in a humidified tissue culture incubator at 10% CO₂ and 37°C, and passaged every 2-3 days. Cells are either cultured in the presence or absence of Norrin.

ES cells are differentiated into embroyid bodies A total of 60 aliquots (30 µl) of ES cell suspension containing 2.5 ×10³ cells are plated as individual drops onto 100 mm² bacteriological dishes (Valmark Inc., Canada). The plates are then gently inverted into hanging drops and the cells incubated. The CEB are cultivated for 40-45 h, and then the dishes are turned right side up and flooded with 10 ml of ES culture media without LIF. The culture media is replaced every three days. The attached cultures of CEB are transferred at day 4 or day 5 to gelatin-coated tissue culture plates or gelatin-coated cover glasses, onto which the CEB attaches, flattens and spreads. Cells are fixed and stained for PECAM-1 expression as described by Ng, Y, et al.

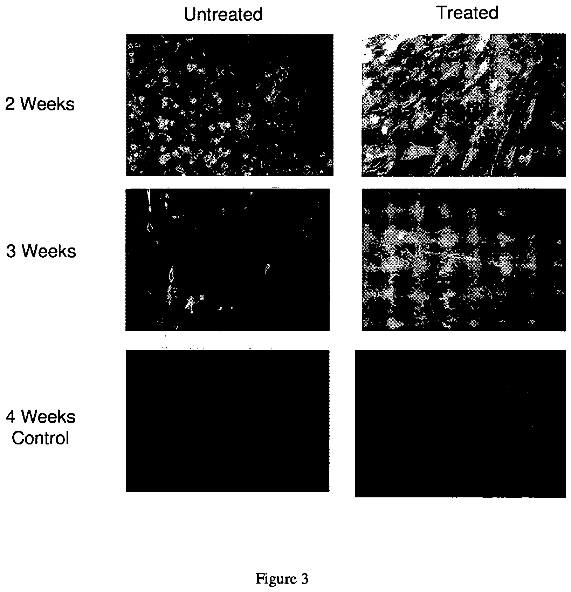

PECAM-1 staining (red) reveals that Norrin induces earlier vascular channel development than non-norrin treated cells with vascular channels appearing at week 2 in Norrin treated groups and not readily apparent in untreated until week 4 (FIG. 3).

### Example 8

Treatment of Norrie disease eyes with recombinant Norrin promotes improved retinal response. Subjects who have been diagnosed with Norrie disease or are at risk for Norrie disease are exposed to norrin prepared essentially as described in example 1 and administered as described in examples 2 or 3. Administration is performed either a single time, or repeated weekly depending on the delivery mechanism used. At weeks 1, 2, 4, and 20 subjects are analyzed for improved retinal physiological activity using electroretinograms by methods known in the art or illustratively as described by Wu, W, et al., Molecular Vision, 2004; 10:93-102. Subjects treated with Norrin demonstrate improved a- and b-wave measurements at all time points tested.

Various modifications of the present invention, in addition to those shown and described herein, will be apparent to those skilled in the art of the above description. Such modifications are also intended to fall within the scope of the appended claims.

Patents and publications mentioned in the specification are indicative of the levels of those skilled in the art to which the invention pertains.

The foregoing description is illustrative of particular embodiments of the invention, but is not meant to be a limitation upon the practice thereof. The following claims, including all equivalents thereof, are intended to define the scope of the invention.

### REFERENCE LIST

1. Connolly, SE, et al, Microvasc Res, 1988; 36:275-290
2. Provis, JM, Prog Retin Eye Res, 2001; 20:799-821
3. Fruttiger, M, Invest Ophthalmol Vis Sci, 2002; 43:522-527
4. Ohlmann, A, et al, J Neurosci, 2005; 25:1701-1710
5. Meitinger, T, et al, Nat Genet, 1993; 5:376-380
6. Berger, W, et al, Hum Mol Genet, 1996; 5:51-59
7. Xu, Q, et al, Cell, 2004; 116:883-895
8. Clevers, H, Curr Biol, 2004; 14:R436-437
9. Nichrs, C, Dev Cell, 2004; 6:453-454
10. Willert K, and Nusse R, Curr Opin Genet Dev, 1998; 8:95-102.
11. Inoue, T, et al, Stem Cells, 2006; 24:95 -104
12. Rhem, HL, et al, J Neurosci, 2002; 22:4286-4292
13. Black, GC, et al, Hum Mol Genet, 1999; 8:2031-2035
14. Robitaille, J, et al, Nature Genet, 2002; 32:326-330
15. Kondo, H, et al, Br J Opthalmol, 2003; 87:1291-1295
16. Toomes, C, et al, Am JHum Genet, 2004; 74:721-730
17. Planutis, K, et al, BMC Cell Biology, 2007; 8:12
18. Perez-Vilar, J and Hill, RL, J Biol Chem, 1997; 272: 33410-33415
19. Smallwood, PM, et al, J Biol Chem, 2007: 282:4057-4068
20. Shaw, LC, et al, Gene Therapy, 2006; 13:752-760
21. Dai, C, et al, J Virology, 2004; 78:6209-6221
22. Nakhai, H, et al, Development, 2007; 134:1151-1160
23. Jiang, C, et al, Mol Vis, 2007; 13:1783-92
24. Fu, K, et al, J Pharm Sci, 2003; 92:1582-91
25. Lin, S, et al, Molecular Vision 2009; 15:26-37

### REFERENCE SEQUENCES

SEQ ID NO 1: Norrin (Homo sapiens) protein sequence 
1 
61 
121 
SEQ ID NO 2: Norrin (Mus musculus) protein sequence 
1 mrnhvlaasi smlsllaimg dtdsktdssf lmdsqrcmrh hyvdsishpl ykcsskmvll

61 arceghcsqa srseplvsfs tvlkqpfrss chccrpqtsk lkalrlrcsg gmrltatyry

121 ilschceecs s
SEQ ID NO 3: Norrin (Rattus norvegicus) protein sequence 
1 mrnhvlaasi smlsllaimg dtdsktdssf lmdsqrcmrh hyvdsishpl ykcsskmvll
61 arceghcsqa srseplvsfs tvlkqpfrss chccrpqtsk lkalrlrcsg gmrltatyry
121 ilschceecs s
SEQ ID NO 4: NDP gene (Homo sapiens) 
1 attcccattt gctagtgcgc tgctgcggcc gcacgcctga ttgatatatg actgcaatgg
61 cacttttcca tttgacattc tctctctctc tctccctctc tctctctccc tctctctctc
121 cctctctctc tctccctgtg tcgcttaaac aacagtccta acttttgtgt gttgcaaata
181 taaaaggcaa gccatgtgac agagggacag aagaacaaaa gcatttggaa gtaacaggac
241 ctctttctag ctctcagaaa agtctgagaa gaaaggagcc ctgcgttccc ctaagctgtg
301 cagcagatac tgtgatgatg gattgcaagt gcaaagagta agacaaaact ccagcacata
361 aaggacaatg acaaccagaa agcttcagcc cgatcctgcc ctttccttga acgggactgg
421 atcctaggag gtgaagccat ttccaatttt ttgtcctctg cctccctctg ctgttcttct
481 agagaagttt ttccttacaa caatgagaaa acatgtacta gctgcatcct tttctatgct
541 ctccctgctg gtgataatgg gagatacaga cagtaaaacg gacagctcat tcataatgga
601 ctcggaccct cgacgctgca tgaggcacca ctatgtggat tctatcagtc acccattgta
661 caagtgtagc tcaaagatgg tgctcctggc caggtgcgag gggcactgca gccaggcgtc
721 acgctccgag cctttggtgt cgttcagcac tgtcctcaag caacccttcc gttcctcctg
781 tcactgctgc cggccccaga cttccaagct gaaggcactg cggctgcgat gctcaggggg
841 catgcgactc actgccacct accggtacat cctctcctgt cactgcgagg aatgcaattc
901 ctgaggcccg ctgctgtgtg tggcttctgg atgggacaac tgtagaggca gttcgaccag
961 ccagggaaag actggcaaga aaagagttaa ggcaaaaaag gatgcaacaa ttctcccggg
1021 actctgcata ttctagtaat aaagactcta catgcttgtt gacagagaga gatactctgg
1081 gaacttcttt gcagttccca tctcctttct ctggtacaat ttcttttggt tcattttcag
1141 attcaggcat tttccccctt ggctctcaat gctgtttggg tttccaacaa ttcagcatta
1201 gtgggaaaaa gtgggccctc atacacaagc gtgtcaggct gtcagtgttt ggtgcacgct
1261 ggggaagaat ttactttgga aagtagaaaa gcccagcttt tcctgggaca tcttctgtta
 
1321 ttgttgatgt ttttttttac cttgtcattt tggtctaagg ttgccattgc tgctaaaggt
1381 taccgatttc aaagtccaga taccaagcat gtggatatgt ttagctacgt ttactcacag
1441 ccagcgaact gacattaaaa taactaacaa acagattctt ttatgtgatg ctggaactct
1501 tgacagctat aattattatt cagaaatgac tttttgaaag taaaagcagc ataaagaatt
1561 tgtcacagga aggctgtctc agataaatta tggtaaaatt ttgtaaggga gcagactttt
1621 aaagacttgc acaaatacgg atcctgcact gactctggaa aaggcatata tgtactagtg
1681 gcatggagaa tgcaccatac tcatgcatgc aaattagaca accaagtatg aatctatttg
1741 tgggtgtgct atagctttag ccgtgtcacg ggcatcattc tctaatatcc acttgtccat
1801 gtgaaacatg ttgccaaaat ggtggcctgg cttgtcttct gaacgtttgg ttcaaatgtg
1861 ttttggtcct ggaggctcaa attttgagtt attcccacgt tttgaaataa aaagagtata
1921 ttcaaaaaaa aaaaa
SEQ ID NO 5: NDP gene (Mus musculus) 
1 gatgctccat ggaaaagatc cgagcagtgg gcagaggctg tgagtccccg ataacgagag
61 cgcctaacat ttccgtggta ttcccatttg ctagagcgct gctgcggcca cacgcctgat
121 tgatatatgc ctgcaatggc acttttccat ttgacactct ccctctctct ctccctctct
181 ctccctctct ctctctccct ctctctccct ctctctccct gggtcgctta aacaacagtc
241 ctaacttttg tgtgttgcaa atataaaagg caagccatgt gacagaggga cagaagaaca
301 aaagcatttg gaagtaacag gacctctttc tagctctcag aaaagtctga gaagaaagga
361 gccctgcgtt tcccccaagc tgtgcagcac atactgctgt gatcggttgc aagtgaaaga
421 agcaagagaa attcctgcat acaaaggaca atgacaacca gaaagcggca gccctatcct
481 gctctggcct tgaaagggac tggatcctag gaggtggcag catttccaat ctattgtcct
541 ctgtctccct ctgctgtttt tctggaggag tttcccttta caacaatgag aaatcatgta
601 ctagctgcat ccatttctat gctctccctg ctggccataa tgggagatac agacagcaaa
661 acagacagtt catttctgat ggactctcaa cgctgcatga gacaccatta tgtcgattct
721 atcagtcacc cactgtacaa atgtagctca aagatggtgc tcctggccag atgtgagggg
781 cactgcagcc aggcatcacg ctctgagccc ttggtgtcct tcagcactgt cctcaagcaa
841 cctttccgtt cctcctgtca ctgctgccga ccccagactt ccaagctgaa ggctctgcgt
901 ctgcgctgct cagggggcat gcgacttact gccacttacc ggtacatcct ctcctgtcac
961 tgtgaggaat gcagctcctg agacttgctg atgattggct ttctgactgg cacaaccaca
1021 ggagcagttc aacctgccag agacggactg gcaagaaaag agttaaggca gataaagatg
1081 gagcaagtcc cataggattt tgcatattct tgtcctaaag actcaatgtg cttttgacag
1141 aaagtgactc tgggaacttg cttttcattc ccatctcctt tccctggaag aatttctttt
1201 ggttacttta cagattcagg catttcccct gttggctcta attgtggttt gggtgcctga
1261 cagctctgca ttagtgggaa aatgtggggc cctgtgcaat agcatgtcag gctgttccta
1321 tttggtgcat attagggaaa attttaccta actctcctta ggagatcttt gcttgttgtt
1381 tcccctggtc attttggtct aagatttgcc tctaaagttt cctggtttca agatctggac
1441 acccagtcca tggatgttta gtgaggctta ctcacagcca gctaactgct actaaaataa
1501 ctaacacatg ggttctttta tgtgacagcg ggactcctga ccactatagt aattattcag
1561 aagtgactga ggggatataa atgtggcaga ggaatttata atctgaagcc ttttgtgagg
 
1621 aagcaggctt tcacacatac acactcaggt ggatcctgca ctgactctgg agaaggcata
1681 cattatactt ggtgtggaga acacaccata ctcataattg agcattagtc aagcatgtag
1741 gaatctactt gtgggtgtgc aatagcttca gccatatctt agctatatcc acctgtctat
1801 gtgaagcttg ttgccgtagt ggtggcccga cttattgtct gaaatttttg tttcaatata
1861 tttttggtcc tcgaagctca aattttgaag tctccccatg ttttcaaata aaaatagagt
1921 accttcaaaa aaaaaaaaaa aaaaaaaaaa aa
SEQ ID NO 6: NDP gene (Rattus norvegicus)

1 cagtgggcag aggctgtgag tccccgataa agagagcgcc taacatttcc gtggtattcc
61 catttgctag tgcgctgctg cggccacacg cctgattgat atatgcctgc aatggcactt
121 ttccatttga cactctccct ctctctccct ctccctctct ctccctctct ctctctccct
181 ctctctccct gggtcgctta aacaacagtc ctaacttttg tgtgttgcaa atataaaagg
241 caagccatgt gacagaggga cagaagaaca aaagcatttg gaagtaacag gacctctttc
301 tagctctcag aaaagtctga gaagaaagga gccctgcgtt tcccccaagc tgtgtagcag
361 gcactgcggt gatggtgatc ggttgcaagt gaaagaagca agacaaactc ctgcatacaa
421 aggacaatga caaccagaaa acggcagccc catcctgccc tggccttgaa aggattgga
481 tcctaggagg tggcagcatt tcagatctat tgtcctctgt cgccctctgc tgttcttctg
541 gagaagtttc cccttacaac aatgagaaat catgtactag ctgcatccat ttctatgctc
601 tccctgctgg ccataatggg agatacagac agcaaaacag acagctcgtt cctgatggac
661 tctcaacgct gcatgaggca ccattatgtc gattctatca gtcacccatt gtacaaatgt
721 agctcaaaga tggtgctcct ggccagatgt gaggggcact gcagccaggc atcacgctct
781 gaacccttgg tgtccttcag cactgtcctc aagcaacctt tccgttcctc ctgtcactgc
841 tgccgacccc agacttccaa gctgaaggct ctgcgtctgc gttgctcagg gggcatgcga
901 cttactgcca cttaccggta catcctctcc tgtcactgtg aggaatgcag ctcctgaggc
961 ttgctgatga ttggctttct ggctggcaca accacaggag caattcaacc tgctagagaa
1021 ggactggcaa gaaaagagtt aaggcagata aagatggagc aagtaccaca ggattgtgca
1081 tactctagtc ctaaagactc gatgtgcttt caacagaaag tgactctggg agcttgctct
1141 tcattccctt ctcctttctg tggaagaatt tcttttggtt acttttcaga ttcaggcatt
1201 tccccttttg gctctgattg tggtttgggt tcctgacagc tctgtattag tgggaaaatg
1261 tggggccctg tgcaagagca cgtcaggtgg tccctatttg gtgcatatta gggaagaatt
1321 taccgaagtc tccttcagtt gttgtttccc cttgttgttt tggtctaaga tttgcctcta
1381 aaggttccca gtttcaaggt ctggacacca agtccatgga tatgtttaga gaggtttact
1441 cacagccagc taactgctac taaataacta acacacgggt tctttcatgt gacagcggga
1501 ctcctgacca ctatagttgt tattcagaaa tgacttaggg gatataaagg tggcagaagg
1561 aatttataat ctgaagactt ttgcaggtaa atgatggtaa acttttgtga ggaagcaggc
1621 tttcacacat atacactcag atggatcctg tcttggctct ggagaaggca tacattacat
1681 gtggcatgga gaacacatca tactcttgat tgagcattag tcaagcatta gtctacttgt

1741 gggtgtgcaa tagcttcagc catatcttga atgtcattcc ctaacatcca cctgtctatg
1801 tgaagtttgt tgccataatg gtggcccggc ttatcctctg aaaactttgt tttcaatata
1861 cttttagtcc ttgaagctca aattttgaat ctccccatgt tttgaaataa aaatagagta
1921 ccttc

## Claims

1. A Norrin compound for use in the treatment of a pathological condition of the retina, wherein the Norrin compound is a nucleotide sequence encoding a Norrin protein, a cell transfected with a nucleotide sequence encoding a Norrin protein and comprising said nucleotide sequence, or a Norrin protein wherein the Norrin protein is selected from the group consisting of a polypeptide having the sequence of SEQ ID NO: 1, SEQ ID NO: 2, SEQ ID NO: 3, an oligomeric complex comprising a Norrin protein, and a mutant form of Norrin protein, wherein the mutant form is selected from the group of consisting of the R64E mutant, the T27A, S28A and S29A mutant; the P36A, R37A and R38A mutant and the H127A, E129A and E130A mutant of the polypeptide of SEQ ID NO:1, wherein the retinal disease is selected from the group consisting of vitreoretinopathy, retinopathy of prematurity, and familial exudative vitreoretinopathy.

2. The Norrin compound for use according to claim 1 wherein the nucleotide sequence encoding a Norrin protein is a gene delivery vector.

3. The norrin compound for use according to any previous claim wherein the compound is to be administered intraocularly, by topical application to the eye, by intraocular injection, local by insertion or injection into the tissue surrounding the eye, systemically through an oral route, or by subcutaneous, intravenous or intramuscular injection.

4. The norrin compound for use according to claim 3 wherein the intraocular injection is into the vitreous space or subretinally.

## Patentansprüche

1. Norrin-Stoff zur Verwendung in der Behandlung eines pathologischen Zustands der Netzhaut, wobei der Norrin-Stoff eine Nukleotidsequenz ist, welche ein Norrin-Protein kodiert, eine Zelle transfiziert mit einer Nukleotidsequenz, welche ein Norrin-Protein kodiert und die Nukleotidsequenz umfasst oder ein Norrin-Protein, wobei das Norrin-Protein ausgewählt wird aus der Gruppe bestehend aus einem Polypeptid, welches die Sequenz von SEQ ID NO: 1, SEQ ID NO: 2, SEQ ID NO: 3 hat, einem oligomeren Komplex umfassend ein Norrin-Protein und einer Mutationsform des Norrin-Proteins, wobei die Mutationsform ausgewählt wird aus der Gruppe bestehend aus der R64E Mutante, der T27A, S28A und S29A Mutante; der P36A, R37A und R38A Mutante und der H127A, E129A und E130A Mutante des Polypeptids der SEQ ID NO: 1, wobei die Netzhauterkrankung ausgewählt wird aus der Gruppe, die aus Vitreoretinopathie, Retinopathie von Frühgeborenen und familiärer exsudativer Retinopathie besteht.

2. Norrin-Stoff zur Verwendung nach Anspruch 1, wobei die Nukleotidsequenz, welche ein Norrin-Protein kodiert, ein Gentransfer-Vektor ist.

3. Norrin-Stoff zur Verwendung nach jedem vorhergehenden Anspruch, wobei der Stoff intraokular, mittels topischer Anwendung am Auge, mittels intraokularer Injektion, lokal mittels Einbringen oder Injektion in das das Auge umgebende Gewebe, systemisch über einen oralen Weg oder mittels subkutaner, intravenöser oder intramuskulärer Injektion zu verabreichen ist.

4. Norrin-Stoff zur Verwendung nach Anspruch 3, wobei die intraokulare Injektion in den Glaskörperraum oder subretinal erfolgt.

## Revendications

1. Un composé Norrine pour une utilisation dans le traitement d'une affection pathologique de la rétine, où le composé Norrine est une séquence nucléotidique codant pour une protéine Norrine, une cellule transfectée avec une séquence nucléotidique codant pour une protéine Norrine et comprenant ladite séquence nucléotidique, ou une protéine Norrine où la protéine Norrine est sélectionnée parmi le groupe constitué d'un polypeptide comprenant la séquence SEQ ID NO: 1, SEQ ID NO: 2, SEQ ID NO: 3, un complexe oligomérique comprenant une protéine norrine, et une forme mutante de protéine Norrine, où la forme mutante est sélectionnée parmi le groupe constitué du mutant R64E, du mutant T27A, S28A et S29A ; du mutant P36A, R37A et R38A et du mutant H127A, E129A et E130A du polypeptide de la SEQ ID NO: 1, où la maladie rétinienne et sélectionnée parmi le groupe constitué de la vitréorétinopathie, la rétinopathie de la prématurité, et la vitréorétinopathie exsudative familiale.

2. Le composé Norrine pour une utilisation selon la revendication 1, où la séquence nucléotidique codant pour une protéine Norrine est un vecteur d'apport de gènes.

3. Le composé Norrine pour une utilisation selon l'une quelconque des revendications précédentes, où le composé Norrine doit être administré par voie intraoculaire, par application topique sur l'oeil, par injection intraoculaire, localement par insertion ou injection dans le tissu autour de l'oeil, systématiquement par voie orale, ou par injection sous-cutanée, intraveineuse ou intramusculaire.

4. Le composé Norrine pour une utilisation selon la revendication 3, où l'injection intraoculaire est effectuée dans l'espace vitréen ou de façon sous-rétinienne.
